# EUROPÄISCHE PATENTSCHRIFT

(11) **EP 2 594 210 B1**
(45) Veröffentlichungstag und Bekanntmachung des Hinweises auf die Patenterteilung: **26.03.2014**
(21) Anmeldenummer: 12007670.8
(22) Anmeldetag: 13.11.2012
(51) Int. Cl.: A61B 17/28, B26B 13/28

(54) **Medizinisches Instrument**
Medical instrument
Instrument médical

(30) Priorität: 17.11.2011 DE 202011107977 U
(43) Veröffentlichungstag der Anmeldung: 22.05.2013
(73) Patentinhaber: Innovations Medical GmbH, 78532 Tuttlingen (DE)
(72) Erfinder: Kreidler, Winfried, 78532 Tuttlingen (DE)
(74) Vertreter: Binner, Bernhard

(56) Entgegenhaltungen:
- DE-C- 59 030
- DE-C- 66 487
- DE-U1- 29 712 016
- DE-U1-202010 010 843

## Beschreibung

Die Erfindung betrifft ein scherenartiges oder zangenartiges medizinisches Instrument mit einem ersten und einem zweiten Handhebel, welche jeweils einen Handgriff und ein Werkzeugteil aufweisen und welche über einen mit einem radial erweiterten Kopfteil versehenen Lagerzapfen schwenkbar miteinander in Verbindung stehen, wobei der erste Handhebel im Bereich zwischen seinem Handgriff und seinem Werkzeugteil eine Führungsplatte bildet und der zweite Handhebel im Bereich zwischen seinem Handgriff und seinem Werkzeugteil eine Lagerplatte bildet, in deren Bereich der Lagerzapfen feststehend angeordnet ist, wobei die Führungsplatte zur schwenkbaren Aufnahme des Lagerzapfens einen als sich in Längsrichtung der Führungsplatte erstreckendes Langloch ausgebildeten Durchbruch aufweist und wobei sich die Führungsplatte und die Lagerplatte im normalen Betriebszustand des Instrumentes flächig berühren und, wobei die Lagerplatte auf ihrer der Führungsplatte zugewandten Kontaktfläche eine im Bereich des Lagerzapfens angeordnete, quer zur Lagerplatte verlaufende Quernut aufweist, deren Tiefe zumindest der Tiefe der ersten Vertiefung des Langloches entspricht und deren Breite zumindest der Breite der Führungsplatte entspricht und, dass der erste Handhebel mit seiner Führungsplatte in eine mit der Quernut fluchtende Schwenkposition bringbar und in Richtung des Lagerzapfens in die Quernut soweit verstellbar ist, dass das Kopfteil des Lagerzapfens mit der ersten Vertiefung des Langloches außer Eingriff gelangt.

In der DE 20 2010 010 843.8 sind verschiedene, als medizinische Handwerkzeuge bezeichnete Instrumente der gattungsgemäßen Art beschrieben, bei welchen zwei Handhebel vorgesehen sind, die in einem mittleren Bereich jeweils eine Lagerplatte bilden. Dabei kann hier eine der Lagerplatten als Führungsplatte im gattungsgemäßen Sinne angesehen werden.

Im normalen Betriebszustand liegen die eben ausgebildete Lagerplatte und die ebenfalls eben ausgebildete Führungsplatte flächig aufeinander. Dies hat zur Folge, dass manche Stellen, insbesondere im Bereich des die beiden Handhebel bzw. die Lagerplatte und die Führungsplatte miteinander verbindenden Lagerzapfens, maschinell nicht vollständig und zuverlässig gereinigt werden können. Diese Bereiche um den Lagerzapfen der flächig aufeinander liegenden Lagerplatte und Führungsplatte sind für die Reinigungsstrahlen einer Reinigungsflüssigkeit in einer Reinigungsanlage nicht unmittelbar zugänglich, sondern verdecken sich auch bei "geöffnetem" Instrument zumindest teilweise gegenseitig.

Um nun diese aufeinander liegenden Flächen der Lagerplatte und der Führungsplatte für eine Reinigungsflüssigkeit zugänglich zu machen, ist beim Gegenstand der DE 20 2010 010 843.8 vorgesehen, dass der Lagerzapfen eine der beiden Lagerplatten (Führungsplatte) durchragt und in seinem die Führungsplatte durchragenden Endbereich mit einem radial erweiterten Ringflansch versehen ist. Dieser Ringflansch soll zur Lagerplatte einen Abstand aufweisen, welcher zumindest der doppelten Dicke der auf dem Lagerzapfen gelagerten Führungsplatte entspricht. Damit soll erreicht werden, dass der zweite Handhebel in einer maximalen (geöffneten) Schwenklage zum ersten Handhebel entlang des Lagerzapfens in eine Lage verschiebbar ist, in welcher die einander gegenüber liegenden Kontaktflächen der abgeflachten Lagerplatte und der Führungsplatte frei liegen.

Durch diese Ausgestaltung lässt sich ein derart gestaltetes medizinisches Instrument (Handwerkzeug) in eine Öffnungsstellung bringen, in welcher die normaler Weise kritischen Bereiche um den Lagerzapfen freigelegt sind. Damit sind diese Bereiche von einem Reinigungsstrahl einer Reinigungsflüssigkeit erfassbar und können zuverlässig gereinigt werden. Dementsprechend sind die beiden Handhebel in eine geöffnete Schwenkstellung bringbar, in welcher es möglich ist, die Lagerplatte und die Führungsplatte der beiden Handhebel relativ zueinander in Richtung des Lagerzapfens zu verschieben, ohne dass das Handwerkzeug auseinander fallen kann. Zu diesem Zweck ist der Lagerzapfen feststehend mit der ersten Lagerplatte des ersten Handhebels verbunden. Die zweite Lagerplatte (Führungsplatte) des zweiten Handhebels ist über ihre Lagerbohrung (Durchbruch) auf dem Gelenkzapfen relativ zur ersten Lagerplatte bzw. zum ersten Handhebel schwenkbar aufgenommen.

Um nun sicherzustellen, dass die zweite Lagerplatte (Führungsplatte) in der geöffneten Schwenkstellung nicht vom Lagerzapfen abgezogen werden kann, oder versehentlich von diesem abrutscht, weist dieser Lagerzapfen an seinem freien Ende einen radial vorstehenden Ringflansch auf, der beim Verschieben der zweiten Lagerplatte (Führungsplatte) entlang des Lagerzapfens als eine Art Anschlag dient. Um sicherzustellen, dass die Lagerplatte und die Führungsplatte für die Zugänglichkeit mit Reinigungsflüssigkeit einen ausreichenden Abstand voneinander aufweisen, ist der Abstand zwischen der ersten Lagerplatte, welche den feststehenden Gelenkzapfen aufnimmt und dem Ringflansch zumindest doppelt so groß wie die Dicke der Führungsplatte gewählt.

Um die beiden Handhebel in ihrer normalen Betriebsstellung, also bei aufeinander liegender Führungsplatte und Lagerplatte zu sichern, ist im Randbereich der Lagerplatte sowohl werkzeugseitig als auch handgriffseitig jeweils ein Sicherungssteg vorgesehen, welche zur Lagerplatte jeweils einen Abstand aufweisen, welcher etwa der Dicke der Führungsplatte entspricht. Dadurch ergibt sich zwischen dem jeweiligen Sicherungssteg und der Lagerplatte eine Art Aufnahmenut, in welche die Führungsplatte im geschlossenen Zustand des Handwerkzeuges (medizinisches Instrument) schwenkbeweglich eingreift. Durch diese Sicherung mittels der beiden Sicherungsstege können somit die Lagerplatte und die Führungsplatte nicht entlang des Lagerzapfens verschoben werden, so dass die normale Betriebsstellung dieses medizinischen Instrumentes mit den sich berührenden Lagerplatte und Führungsplatte gesichert ist. Dabei ist dieses medizinische Instrument beispielhaft als Zange ausgebildet.

Nachteilig bei dieser Konstruktion ist, dass in normaler Betriebsstellung aufgrund der axialen Länge des Lagerzapfens, dieser mit seinem radial erweiterten Ringflansch über die Führungsplatte hinaus steht, was im Betrieb bzw. bei der Handhabung dieses medizinischen Instrumentes störend ist. Außerdem besteht hier die Gefahr, dass beim Verschieben der Führungsplatte entlang des Lagerzapfens beispielsweise Gewebeteile zwischen dem radial erweiterten Ringflansch und der Führungsplatte eingeklemmt werden, wodurch die Verstellung der Führungsplatte zur Lagerplatte entlang des Lagerzapfens behindert werden kann. Um ein solches Einklemmen zu verhindern, kann zusätzlich auf der Führungsplatte ein umlaufender, über die Führungsplatte vorstehender Ringsteg vorgesehen sein, welcher in der normalen Betriebsstellung, also bei aufeinander liegender Führungsplatte und Lagerplatte, zumindest annähernd bündig mit dem radial erweiterten Ringflansch abschließt. Dies jedoch hat wiederum zur Folge, dass der Ringsteg seitlich über die Führungsplatte vorsteht, was bei der Handhabung dieses Handwerkzeuges (medizinisches Instrument) störend sein kann.

Des weiteren ist bei diesem medizinischen Instrument (Handwerkzeug) vorgesehen, dass die voneinander beabstandete Position der Lagerplatte und der Führungsplatte durch die Sicherungsstege fixierbar ist. Hierzu werden die Lagerplatte und die Führungsplatte in ihrem voneinander beabstandeten Zustand aus ihrer vollständig geöffneten Schwenkstellung teilweise in Richtung ihrer geschlossen Stellung "zurück" verstellt, so dass die Führungsplatte außenseitig auf den Sicherungsstegen zur Anlage kommt. Damit wird aber wiederum ein Teil der zur Lagerplatte hin weisenden Kontaktfläche wieder abgedeckt, so dass dessen Reinigung nicht optimal durchgeführt werden kann.

Weiter ist aus der DE 66 487 C ein als zerlegbare Zange, Schere oder dgl. bezeichnetes Instrument bekannt, welches die Merkmale des Oberbegriffes des Anspruches 1 aufweist und für medizinische Zwecke genutzt werden kann. Bei dieser Konstruktion bildet das Langloch in seinem dem Werkzeugteil gegenüber liegenden Endbereich eine erweiterte, durchgehende Ausrundung. Zur Demontage der beiden Handhebel wird der Lagerzapfen des Kopfteils, nachdem das Kopfteil mit der erste Vertiefung außer Eingriff gebracht wurde, entlang des Langloches verstellt, bis der Lagerzapfen mit seinem Kopfteil im Bereich der Ausrundung angeordnet ist. Die erweiterte Ausrundung ist derart dimensioniert, dass in dieser Position der Lagerzapfen mit seinem Kopfteil durch die Ausrundung hindurch passt und somit das Instrument in dieser Position zerlegt werden kann. Bei dieser Konstruktion muss das Instrument folglich zu Reinigungszwecken stets vollständig zerlegt werden, um die Kontaktflächen der Lagerplatte und der Führungsplatte frei zugänglich zu machen. Werden mehrere solcher Instrumente gleichzeitig einem Reinigungsvorgang zugeführt, so kann anschließend für die Montage eine Zuordnung der ursprünglich zusammen gehörenden Handhebel nicht mehr gewährleistet werden.

Demgemäß liegt der Erfindung die Aufgabe zugrunde, ein medizinisches Instrument der gattungsgemäßen Art derart auszugestalten, dass die genannten Nachteile sicher vermieden sind.

Die Aufgabe wird erfindungsgemäß zusammen mit den Merkmalen des Oberbegriffes des Anspruches 1 dadurch gelöst, dass das Langloch im Randbereich seines in Längsrichtung dem Werkzeugteil gegenüber liegenden Endbereichs eine zweite erweiterte Vertiefung aufweist und, dass das Kopfteil des Lagerzapfens wahlweise in der ersten oder der zweiten Vertiefung aufnehmbar ist und, dass die erste Vertiefung eine geringere Tiefe aufweist als die zweite Vertiefung und, dass das Kopfteil des Lagerzapfens durch eine Verstellung des zweiten Handhebels mit seiner Lagerplatte und dem Lagerzapfens entlang des Langloches mit der zweiten Vertiefung des Langloches in Überdeckung und durch eine Verstellung in Richtung des Lagerzapfens mit der zweiten Vertiefung in Eingriff bringbar ist, ohne dass das Instrument auseinander fallen kann und, dass die Führungsplatte und die Lagerplatte in dieser Position einen Abstand voneinander aufweisen.

Durch die erfindungsgemäße Ausgestaltung wird ein medizinisches Instrument, beispielsweise in Form einer Schere oder Zange, zur Verfügung gestellt, bei welchem die Lagerplatte und die Führungsplatte derart gegeneinander verstellbar sind, dass diese zu Reinigungszwecken einen sicheren Abstand voneinander aufweisen.

Hierzu ist in der Führungsplatte ein Durchbruch in Form eines Langloches vorgesehen, welches im werkzeugseitigen Endbereich und diesem in Längsrichtung der Führungsplatte gegenüber liegenden Endbereich jeweils eine erweiterte Vertiefung aufweist. In diesen beiden Vertiefungen ist das Kopfteil des Lagerzapfens wahlweise aufnehmbar.

Die erste werkzeugseitige Vertiefung weist dabei eine geringere Tiefe auf, als die zweite in Längsrichtung gegenüberliegende zweite Vertiefung. Im normalen Betriebszustand steht das Kopfteil des Lagerzapfens mit der ersten Vertiefung geringerer Tiefe in Eingriff, so dass die Führungsplatte mit ihrem Langloch nicht entlang des Lagerzapfens verstellbar und somit gesichert ist. Um nun die Führungsplatte und die Lagerplatte voneinander zu Reinigungszwecken in Richtung des Lagerzapfens trennen zu können, ist nunmehr vorgesehen, dass die Führungsplatte in einer geöffneten, im Wesentlichen rechtwinklig zur Lagerplatte verlaufenden Schwenkstellung in eine Quernut der Lagerplatte in Richtung des Lagerzapfens verstellt werden kann. Damit wird der Eingriff des Kopfteils des Lagerzapfens mit der ersten Vertiefung aufgehoben. Nun kann die Führungsplatte mit ihrem Langloch entlang des Lagerzapfens soweit verschoben werden, bis das Kopfteil des Lagerzapfens mit der zweiten Vertiefung größerer Tiefe in Überdeckung gelangt. Durch anschließendes Verstellen der Führungsplatte entlang des Lagerzapfens gelangt nun das Kopfteil mit dieser zweiten Vertiefung größerer Tiefe in Eingriff.

Der Stellweg in diese Richtung ist dabei größer als der Stellweg bei der ersten Verstellung, so dass die Führungsplatte und die Lagerplatte nach Ausführung dieser Verstellung und bei vollständiger Aufnahme oder zumindest teilweiser Aufnahme des Kopfteils des Lagerzapfens in der zweiten Vertiefung größerer Tiefe einen Abstand voneinander aufweisen. Damit sind die beiden sich gegenüber liegenden Kontaktflächen der Führungsplatte und der Lagerplatte für eine Reinigungsflüssigkeit unabhängig von einer evtl. anschließenden Veränderung der Schwenkstellung der beiden Handhebel zueinander frei zugänglich, so dass eine Reinigung in einfacher Weise auch maschinell durchführbar ist. Des Weitern ist die Gefahr von Verletzungen erheblich minimiert, da keine Sicherungsstege vorhanden sind und somit kein Einklemmen der Finger zwischen den (nicht vorhandenen) Sicherungsstegen und der Führungsplatte beim Herstellen der normalen Betriebsstellung auftreten kann.

Weitere vorteilhafte Ausgestaltungen der Erfindung sind den weiteren Unteransprüchen entnehmbar.

So kann gemäß Anspruch 2 vorgesehen sein, dass die Tiefe der ersten Vertiefung des Langloches derart bemessen ist, dass das Kopfteil in der normalen Betriebsstellung darin zumindest annähernd vollständig aufgenommen ist. Mit normaler Betriebsstellung ist hier die relative Position der beiden Handhebel gemeint, in welcher diese mit ihren Kontaktflächen aufeinander liegen und manuell gegeneinander zur Ausführung einer Arbeitsaufgabe schwenkbar sind. Durch die Ausgestaltung nach Anspruch 2 ist sichergestellt, dass das Kopfteil des Lagerzapfens nicht "seitlich" störend über das Instrument hinausragt.

Weiter kann gemäß Anspruch 3 vorgesehen sein, dass der Lagerzapfen als Lagerschraube ausgebildet ist, welche mit einem Gewindezapfen in ein Innengewinde der Lagerplatte einschraubbar ist und, dass das Innengewinde zentral in der Quernut der Lagerplatte angeordnet ist. Durch diese Ausgestaltung wird einerseits eine einfache Montage der beiden Handhebel sichergestellt. Andererseits kann die Führungsplatte durch einfaches schwenken, beispielsweise um 90°, relativ zur Lagerplatte mit der Quernut in Überdeckung gebracht werden, da die Quernut symmetrisch zum die Lagerschraube aufnehmenden Innengewinde angeordnet ist.

Durch die Ausgestaltung nach Anspruch 4 wird erreicht, dass das Kopfteil des Lagerzapfens oder der Lagerschraube stets versenkt in der Führungsplatte aufgenommen wird, unabhängig davon, ob dieses sich beim Verstellvorgang im Bereich des Langloches oder am Ende des Stellvorganges im Bereich einer der beiden Vertiefungen befindet. Dazu ist vorgesehen, dass das Langloch der Führungsplatte zwischen den beiden Vertiefungen im Bereich seiner Längskanten jeweils eine seitlich eingesenkt abgesetzte Erweiterung aufweist, über welche die Vertiefungen miteinander in Verbindung stehen und, dass die Vertiefungen mit einer größeren Tiefe ausgestattet sind als die seitliche Erweiterung.

Weiter kann gemäß Anspruch 5 vorgesehen sein, dass die Vertiefungen mit konisch zum Langloch hin geneigt verlaufenden Grundflächen versehen sind und, dass das Kopfteil des Lagerzapfens unterseitig zum Langloch hin eine umlaufende, konische Auflagefläche bildet, mit welcher das Kopfteil passend mit einer der Grundflächen in Eingriff bringbar ist. Durch diese Ausgestaltung wird der Lagerzapfen, sobald dieser mit den Grundflächen einer der Vertiefungen in Eingriff gelangt präzise im entsprechend zugehörigen Endbereich des Langloches ausgerichtet. Des Weiteren kann eine Relativbewegung der Führungsplatte in Richtung des Lagerzapfens durch eine Querverschiebung der Führungsplatte zur Lagerplatte bewirkt werden, da die konischen Grundflächen und die konische Auflagefläche nach Art einer Rampe zusammenwirken. Dies erleichtert insbesondere den Wechsel der Eingriffspositionen des Kopfteils von der einen Vertiefung in die jeweils anderen Vertiefung.

Durch die Ausgestaltung nach Anspruch 6 wird sicher verhindert, dass das Kopfteil des Lagerzapfens in der geöffneten Reinigungsposition aus seinem Eingriff mit der zweiten Vertiefung des Langloches gelangen kann. Aber auch die Eingriffposition des Kopfteils mit der ersten Vertiefung ist insbesondere in der geöffneten Schwenklage der beiden Handhebel gesichert, da sich die auf die Quernut ausgerichtete Führungsplatte nicht unbeabsichtigt in Richtung auf die Lagerplatte bewegen kann. Eine solche Stellbewegung der Führungsplatte zur Lagerplatte hin ist in den normalen nicht auf die Quernut ausgerichteten Arbeitsstellungen der Führungsplatte ausgeschlossen, da die beiden Kontaktflächen der Führungsplatte und der Lagerplatte in jeder anderen Schwenkstellung (zumindest teilweise) flächig aufeinander liegen und somit das Kopfteil des Lagerzapfens sicher in der ersten Vertiefung des Langloches gehalten wird. Zur Sicherung der beiden Eingriffspositionen ist nach Anspruch 6 vorgesehen, dass zwischen der Lagerplatte und der Führungsplatte ein Federelement, insbesondere in Form einer Tellerfeder vorgesehen ist, durch welche das Kopfteil in seiner jeweiligen Eingriffsposition mit einer der beiden Vertiefungen des Langloches gehalten ist.

Um eine Beschädigung des Lagerzapfens bei einer relativen Stellbewegung der Lagerplatte mit ihrem Lagerzapfen entlang des Langloche zu vermeiden ist gemäß Anspruch 7 vorgesehen, dass das Federelement in einer um den Lagerzapfen der Lagerplatte umlaufenden, ringförmigen Einsenkung aufgenommen ist. Damit wird das Federelement im Bereich der Quernut der Lagerplatte unverschieblich auf der Lagerplatte gehalten, so dass sich das Federelement bei der genannten Stellbewegung stets mit dem feststehend mit der Lagerplatte verbundenen Lagerzapfen mitbewegt.

Anhand der Zeichnung werden nachfolgend beispielhaft zwei Ausführungsvarianten der Erfindung näher erläutert. Es zeigt:
- Fig. 1: eine perspektivische Darstellung einer ersten Ausführungsvariante eines als Schere ausgebildeten medizinischen Instrumentes mit einem ersten und einem zweiten Handhebel, welche in Fig. 1 in einer geschlossenen Schwenkstellung dargestellt sind;
- Fig. 2: einen vergrößerten Teilschnitt der Führungsplatte des ersten Handhebels des medizinischen Instrumentes aus Fig. 1;
- Fig. 3: einen perspektivischen Teilschnitt der Lagerplatte des zweiten Handhebels des medizinischen Instrumentes aus Fig. 1;
- Fig. 4: einen vergrößerten perspektivischen Teilschnitt des ersten und des zweiten Handhebels im Bereich der Führungsplatte bzw. der Lagerplatte in einer geöffneten Schwenkstellung;
- Fig. 5: die Darstellung aus Fig. 4, in welcher die Führungsplatte entlang des Lagerzapfens in eine Quernut der Lagerplatte verstellt ist;
- Fig. 6: die Darstellung aus Fig. 5, in welcher die Führungsplatte mit ihrem Langloch entlang des Lagerzapfens verschoben ist;
- Fig. 7: die Darstellung aus Fig. 6, in welcher die Führungsplatte von der Quernut bzw. der Lagerplatte abgehoben ist, wobei in diesem Zustand das Kopfteil des Lagerzapfens voll umfänglich von der zweiten Vertiefung größerer Tiefe des Langloches aufgenommen ist;
- Fig. 8: einen perspektivischen Teilschnitt der Lagerplatte des zweiten Handhebels mit aufgesetzter Tellerfeder sowie eine zweite Ausführungsvariante der Führungsplatte im Bereich des Langloches mit einer um das Langloch umlaufenden erweiterten Einsenkung, in welcher die beiden Vertiefungen in den Endbereichen des Langloches angeordnet sind;
- Fig. 9: eine Seitenansicht eines Lagerzapfens mit einem speziell ausgestalteten Kopfteil;
- Fig. 10: die Führungsplatte und die Lagerplatte der beiden Handhebel im geschlossenen, aufeinander liegenden Zustand, in welchem diese durch den Lagerzapfen aus Fig. 9 mit dessen Kopfteil gesichert sind;
- Fig. 11: eine Schnittdarstellung durch die Führungsplatte und durch die Lagerplatte, wobei sich die Führungsplatte und die Lagerplatte in der aus den Fig. 4 bis 7 erkennbaren, geöffneten Position befinden.

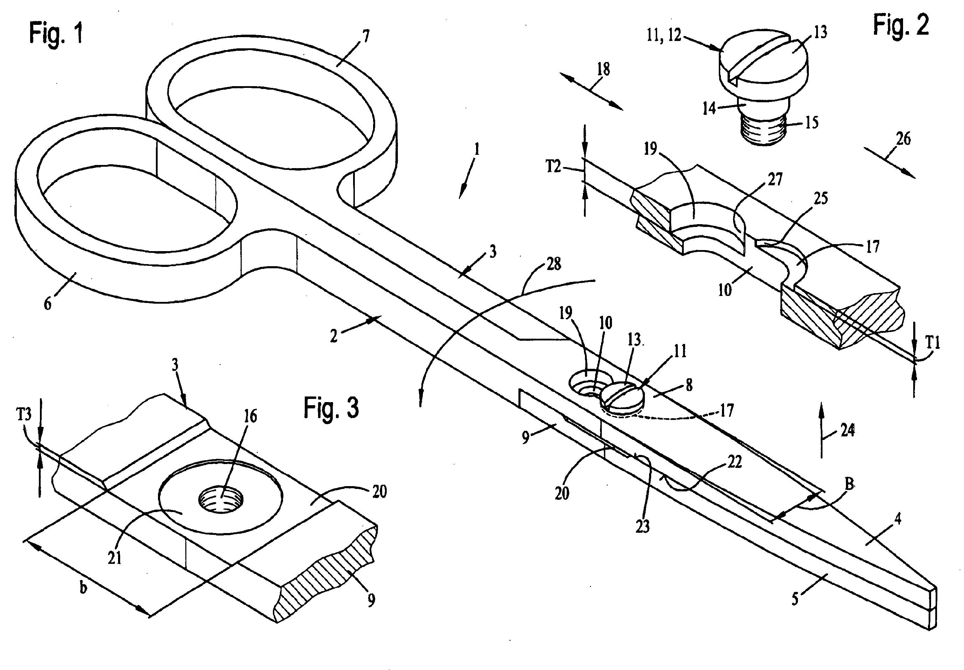

Fig. 1 zeigt eine perspektivische Darstellung eines medizinischen Instrumentes 1, welches bei der dargestellten Ausführungsvariante als Schere ausgebildet ist. Dieses medizinische Instrument 1 besteht aus einem ersten Handhebel 2 und einem zweiten Handhebel 3, welche jeweils ein Werkzeugteil 4 bzw. 5 und ein Griffteil 6 bzw. 7 bilden. Zwischen dem jeweiligen Werkzeugteil 4 bzw. 5 und dem jeweiligen Griffteil 6 bzw. 7 weisen die beiden Handhebel 2 und 3 eine Führungsplatte 8 bzw. eine Lagerplatte 9 auf. Die Führungsplatte 8 ist zur schwenkbaren Lagerung des ersten Handhebels 2 am zweiten Handhebel 3 mit einem Langloch 10 versehen, durch welches ein Lagerzapfen 11 hindurch gesteckt ist.

Wie aus Fig. 2 ersichtlich ist, ist dieser Lagerzapfen 11 bei der dargestellten Ausführungsvariante als Lagerschraube 12 ausgebildet, welche mit einem radial erweiterten Kopfteil 13 versehen ist. An dieses Kopfteil 13 schließt sich nach unten hin ein Lagerzylinder 14 an, an welchem ein nach unten vorstehender, im Durchmesser verjüngt ausgebildeter Gewindezapfen 15 angeordnet ist. Dieser Lagerzapfen 11 bzw. die Lagerschraube 12 ist durch das Langloch 10 hindurch steckbar und in ein Innengewinde 16 (Fig. 3) einschraubbar, welches im Bereich der Lagerplatte 9 des zweiten Handhebels 3 zentral angeordnet ist. Im montierten Zustand wird somit die Führungsplatte 8 über den Lagerzylinder 14 und das Langloch 10 schwenkbar an der Lagerplatte 9 und somit am zweiten Handhebel 3 gelagert.

Wie insbesondere aus Fig. 2 ersichtlich ist, weist das Langloch 10 eine erste Vertiefung 17 auf, welche oberseitig im werkzeugseitigen Endbereich des Langloches 10 angeordnet ist. In Längsrichtung des Doppelpfeils 18 der ersten Vertiefung 17 gegenüber liegenden Endbereich des Langloches 10 ist eine zweite Vertiefung 19 vorgesehen. Wie aus Fig. 2 ersichtlich ist, weist die erste Vertiefung 17 eine geringere Tiefe **T1** auf, welche kleiner ist als die Tiefe **T2** der zweiten Vertiefung 19.

Im montierten Zustand steht das Kopfteil 13 mit der ersten Vertiefung 17 des Langloches 10 der Führungsplatte 8 formschlüssig und drehbar in Eingriff, wie dies andeutungsweise aus Fig. 1 ersichtlich ist.

Aus Fig. 3 ist des Weiteren erkennbar, dass oberseitig auf der Lagerplatte 9 im Bereich des Innengewindes 16 - und somit im Bereich eines montierten Lagerzapfens 11 bzw. einer Lagerschraube 12 - eine quer zur Lagerplatte 9 verlaufende Quernut 20 vorgesehen ist. Die Breite **b** dieser Quernut 20 entspricht dabei wenigstens der Breite **B** der Führungsplatte 8. Die Tiefe **T3** dieser Quernut 20 entspricht zumindest der Tiefe **T1** der ersten Vertiefung 17 des Langloches 10. Weiter ist aus Fig. 3 noch erkennbar, dass die Quernut 20 im Umgebungsbereich des Innengewindes 16 der Führungsplatte 3 eine kreisringförmige Einsenkung 21 aufweist.

Weiter ist aus Fig. 1 ersichtlich, dass die Führungsplatte 8 in diesem dargestellten Betriebszustand des Instrumentes 1 mit ihrer unteren Kontaktfläche 22 eben auf der oberen Kontaktfläche 23 der Lagerplatte 9 aufliegt. Aufgrund des Eingriffes des Kopfteils 13 des Lagerzapfens 11, bzw. der Lagerschraube 12, mit der ersten Vertiefung 17 wird ein Abheben des ersten Handhebels 2 mit seiner Führungsplatte 8 in Richtung des Pfeiles 24 sicher verhindert. Da die Vertiefung 17 mit ihrer inneren Begrenzungskante 25 (Fig. 2) kreisbogenförmig zum Langloch 10 hin gerichtet ist, wird durch diesen Eingriff des Kopfteils 13 in der Vertiefung 17 auch ein Verschieben des ersten Handhebels 2 mit seiner Führungsplatte 8 in Richtung des Pfeiles 26 auch im geöffneten Zustand der beiden Handhebel 2 und 3 sicher verhindert.

In gleicher Weise bildet auch die zweite Vertiefung 19 eine in das Langloch 10 mündende, innere Begrenzungskante 27, wodurch ein Verschieben der Führungsplatte 8 entgegen des Pfeiles 26 relativ zur Lagerplatte 9 verhindert wird, sofern das Kopfteil 13 mit dieser zweiten Vertiefung 19 in Eingriff steht.

Um das Kopfteil 13 mit der ersten Vertiefung 17 außer Eingriff bringen zu können, ist es erforderlich, zunächst die Führungsplatte 8 entgegen des Pfeiles 24 zu verstellen, so dass das Kopfteil 13 nicht mehr mit dieser ersten Vertiefung 17 in Eingriff steht. Um eine solche Stellbewegung in Richtung des Lagerzapfens 11 und somit entgegen des Pfeiles 24 bewirken zu können, ist eine Schwenkbewegung des ersten Handhebels 2 mit seiner Führungsplatte 8 um den Lagerzapfen 11 in Richtung des Pfeiles 28 zu bewirken, um die Führungsplatte 8 mit der Quernut 20 der Lagerplatte 9 in "Überdeckung" zu bringen.

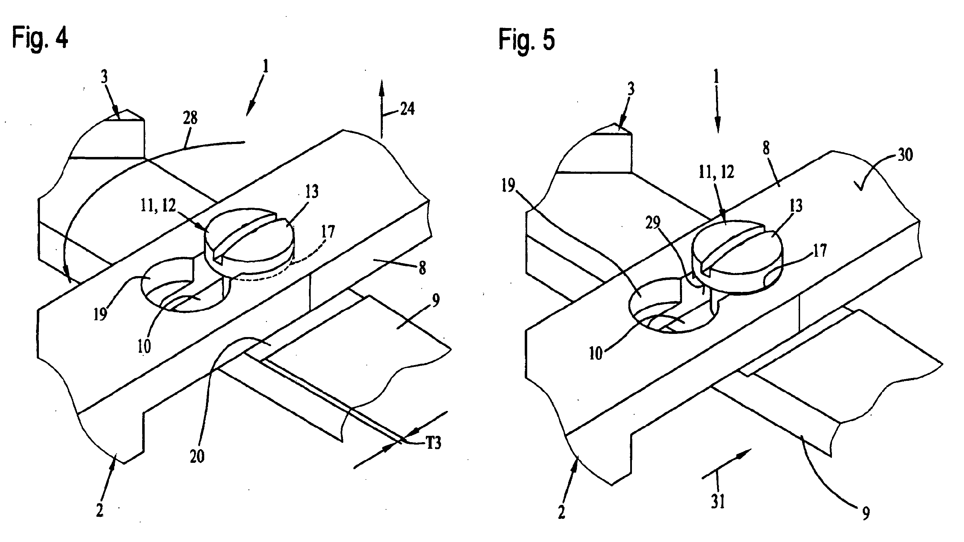

Die Fig. 4 bis 7 zeigen hierzu schematisch die Vorgehensweise, um das Kopfteil 13 des Lagerzapfens 11 bzw. der Lagerschraube 12 von der ersten Vertiefung 17 in die zweite Vertiefung 19 verstellen zu können. Dabei ist in diesen Zeichnungsfiguren 4 bis 7 das Instrument 1 im Bereich der Lagerplatte 9 bzw. der Führungsplatte 8 ausschnittsweise perspektivisch dargestellt.

In Fig. 4 ist der erste Handhebel 2 mit seiner Führungsplatte 8 bereits in Richtung des Pfeiles 28 relativ zum zweiten Handhebel 3 mit seiner Lagerplatte 9 verschwenkt dargestellt. In dieser, beim vorliegenden Ausführungsbeispiel um 90°, gedrehten Position, ist die Führungsplatte 8 auf die Quernut 20 der Lagerplatte 9 ausgerichtet. Das Kopfteil 13 des Lagerzapfens 11 bzw. der Lagerschraube 12 steht mit der ersten Vertiefung 17 noch formschlüssig in Eingriff. Aufgrund dessen, dass die Tiefe **T3** der Quernut 20 zumindest der Tiefe **T1** (Fig. 2) der Vertiefung 17 entspricht, ist die Führungsplatte 8 des Handhebels 2 entgegen des Pfeiles 24 soweit in Richtung des Lagerzapfens 11 bzw. der Lagerschraube 12 verstellbar, bis das Kopfteil 13 mit der Vertiefung 17 außer Eingriff gelangt.

Diesen Zustand zeigt Fig. 5. Es ist erkennbar, dass das Kopfteil 13 mit seiner Unterseite 29 oberhalb der äußeren Oberfläche 30 an der Führungsplatte 9 angeordnet ist, so dass in dieser Position die Führungsplatte 8 in Richtung des Pfeiles 31 mit ihrer Längsnut 10 relativ zum Lagerzapfen 11 bzw. der Lagerschraube 12 verschiebbar ist.

Durch diese Stellbewegung in Richtung des Pfeiles 31 ist das Kopfteil 13 des Lagerzapfens 11 bzw. der Lagerschraube 12 mit der zweiten Vertiefung 19 der Führungsplatte 8 bzw. des Langloches 10 in Überdeckung bringbar.

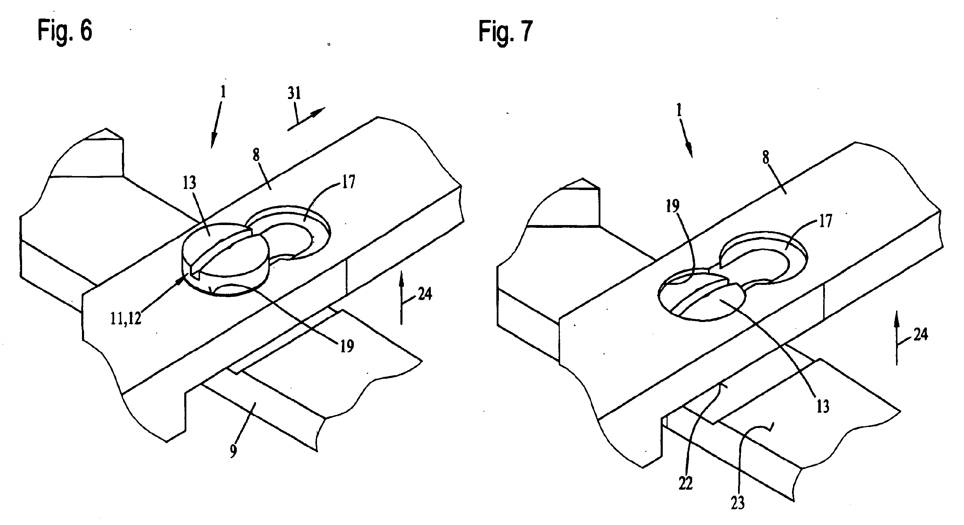

Fig. 6 zeigt hierzu diesen Zustand. Es ist erkennbar, dass die Führungsplatte 8 relativ zur Lagerplatte 9 in Richtung des Pfeiles 31 verschoben ist und die erste Vertiefung 17 freigegeben ist. Das Kopfteil 13 des Lagerzapfens 11 bzw. der Lagerschraube 12 steht mit der zweiten Vertiefung 19 in Überdeckung. Durch anschließendes Anheben der Führungsplatte 8 in Richtung des Pfeiles 24 wird nunmehr das Kopfteil 13 in der zweiten Vertiefung 19 aufgenommen und drehbar aber feststehend gesichert.

Diesen verstellten Zustand zeigt Fig. 7. Aufgrund der größeren Tiefe **T2** (Fig. 2) der Vertiefung 19 im Vergleich zur Tiefe **T1** der ersten Vertiefung 17 vergrößert sich somit der Abstand der beiden Kontaktflächen 22 und 23 der Führungsplatte 8 und der Lagerplatte 9, so dass diese für Reinigungsflüssigkeit frei zugänglich sind. Um das Kopfteil 13 in dieser Eingriffsposition der Fig. 7 zu halten, kann insbesondere im Bereich der Einsenkung 21 der Quernut 20 eine Axialdruckfeder, insbesondere in Form einer Tellerfeder vorgesehen sein.

Aufgrund dieser speziellen Ausführungsvariante wird somit der Abstand zwischen den beiden Kontaktflächen 22 und 23 gehalten, so dass unabhängig von der Schwenkstellung der Führungsplatte 8 zur Lagerplatte 9 diese beiden Kontaktflächen 22 und 23 für Reinigungszwecke stets frei zugänglich sind.

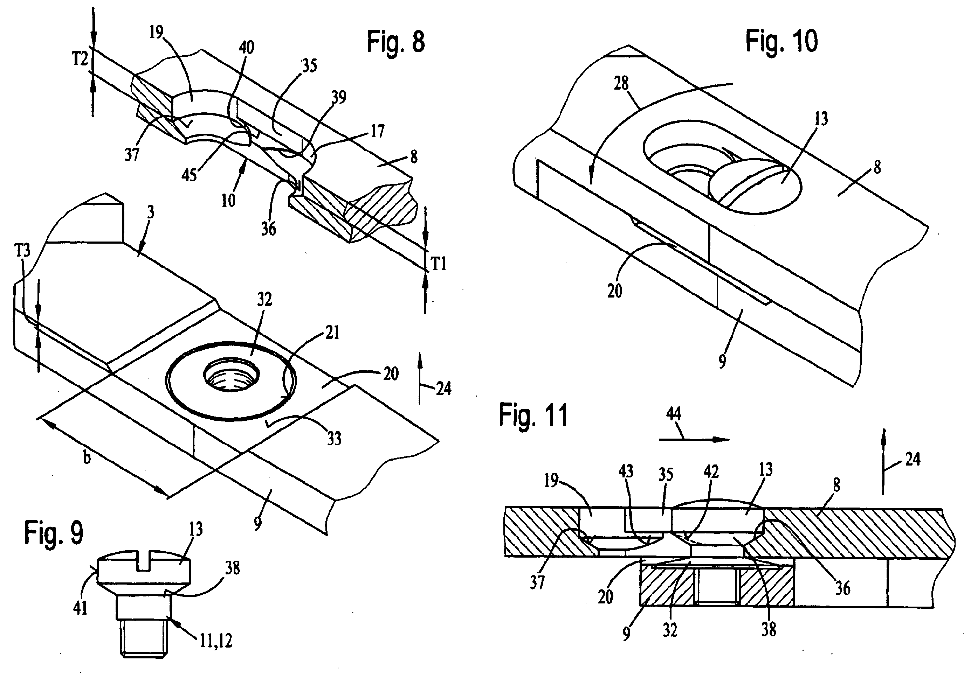

In den Fig. 8 bis 11 ist eine zweite, leicht modifizierte Ausführungsvariante eines medizinischen Instrumentes gezeigt, wobei in diesen Darstellungen lediglich ausschnittsweise die Führungsplatte 8 sowie die Lagerplatte 9 dargestellt sind.

Bei dieser Ausführungsvariante ist insbesondere aus den Fig. 8 und 11 die als Tellerfeder 32 ausgebildete Axialdruckfeder erkennbar. Die Ausgestaltung der Lagerplatte 9 aus Fig. 8 ist identisch mit der Ausgestaltung der Lagerplatte 9 aus Fig. 3, so dass in Fig. 8 auch dieselben Bezugszeichen eingetragen sind. Die Tellerfeder 32 wird mit geringem Spiel in der Einsenkung 21 aufgenommen und steht über deren innere Oberfläche 33 vertikal in Richtung des Pfeiles 24 nach oben vor.

Unterschiedlich zur Ausführungsvariante nach den Fig. 1 bis 7 ist die Ausbildung des Langloches 10 der Führungsplatte 8 mit seinen beiden Vertiefungen 17 und 19. Aus dem Teilschnitt der Fig. 8 der Führungsplatte 8 ist erkennbar, dass die beiden Vertiefungen 17 und 19 über eine seitlich eingesenkte Erweiterung 35 miteinander in Verbindung stehen. Eine solche seitlich eingesenkte Erweiterung 35 ist im Bereich beider oberen Längskanten des Langloches 10 vorgesehen, wobei in Fig. 8 auf Grund der Schnittführung lediglich eine dieser Erweiterungen 35 erkennbar ist. Die beiden Vertiefungen 17 und 19 sind mit einer größeren Tiefe **T1** und **T2** ausgestattet, als die Vertiefungen 17 und 19 der Ausführungsvariante der Längsnut 10 des Ausführungsbeispieles aus den Fig. 1 bis 7. Dabei ist die Tiefe **T1** der ersten Vertiefung 17 kleiner als die Tiefe **T2** der zweiten Vertiefung 19.

Sowohl die Tiefe **T1** als auch die Tiefe **T2** ist größer als die nicht näher bezeichnete Tiefe der eingesenkten Erweiterung 35, so.dass sich für die Eingriffpositionen eines mit einer der Vertiefungen 17 oder 19 in Eingriff stehenden Kopfteils 13 dieselben Funktionsmerkmale ergeben, wie bereits zu der Ausführungsvariante nach der Fig. 1 bis 7 beschrieben. Aufgrund der insgesamt versenkten Anordnung dieser beiden Vertiefungen 17 und 19 sowie der seitlichen Erweiterung 35 ist das Kopfteil 13 des Lagerzapfens 11 bzw. der Lagerschraube 12 auch vollständig versenkt in der ersten Vertiefung 17 mit ihrer geringeren Tiefe **T1** aufnehmbar, wie dies beispielhaft aus Fig. 10 ersichtlich ist.

Ein weiterer Unterschied zu den beiden Vertiefungen 17 und 19 der Ausführungsvariante nach den Fig. 1 bis 7 besteht darin, dass die Grundflächen 36 und 37 konisch ausgebildet sind und leicht schräg zum Langloch 10 hin "abfallen". Dementsprechend ist auch das Kopfteil 13, wie dies aus Fig. 9 ersichtlich ist, als Senkkopf ausgebildet und weist unterseitig eine konische Auflagefläche 38 auf, mit welcher das Kopfteil 13 wahlweise mit der Grundfläche 36 der ersten Vertiefung 17 oder der Grundfläche 37 der zweiten Vertiefung 19 in Kontakt bringbar ist.

Des Weiteren ist aus Fig. 8 ersichtlich, dass im Übergang von den beiden Grundflächen 36 und 37 zur seitlichen Erweiterung 35 ein innerer, in das Langloch 10 mündender Anschlagsteg 39 bzw. 40 ausgebildet ist, mit welchem - je nach Eingriffsposition des Kopfteils 13 - das Kopfteil 13 mit seiner zylindrischen äußeren Mantelfläche 41 im jeweiligen Betriebszustand unverschieblich in Kontakt steht.

Wie insbesondere aus Fig. 11 ersichtlich ist, bilden diese Grundflächen 36 und 37 zur seitlichen Erweiterung 35 hin jeweils eine Art Rampe 42 bzw. 43.

Aufgrund dieser Formgebung der Grundflächen 36 und 37 mit ihren "Rampen" 42 und 43 wird erreicht, dass nach minimalem Niederdrücken der Führungsplatte 8 entgegen des Pfeils 24 durch eine Krafteinwirkung in Richtung des Pfeiles 44 auf die Führungsplatte 8 das Kopfteil 13 an der Rampe 42 "aufgleitet", so dass die Führungsplatte 8 zwangsläufig entgegen der Federkraft der Tellerfeder 32 entgegen des Pfeiles 24 in die Quernut 20 hinein "gepresst" wird. Anschließend gleitet das Kopfteil 13 mit seiner Auflagefläche 38 entlang der aus Fig. 8 erkennbaren Gleitfläche 45 der seitlichen Erweiterung 35, bis das Kopfteil 13 mit der zweiten Vertiefung 19 in Überdeckung gelangt. In dieser Stellposition wird die Führungsplatte 8 zwangsläufig durch die Federkraft der Tellerfeder 32 in Richtung des Pfeiles 24 verstellt, so dass das Kopfteil 13 mit einem entsprechend vergrößerten Abstand der Führungsplatte 8 zur Lagerplatte 9 mit der zweiten Vertiefung 19 in Eingriff gelangt. Zuvor ist es ebenfalls notwendig, die Führungsplatte 8 aus der in Fig. 10 dargestellten Position relativ zur Lagerplatte 9 in Richtung des Pfeiles 28 zu schwenken, um die Führungsplatte 8 mit der Quernut 20 in Überdeckung zu bringen.

Aus den dargestellten Ausführungsbeispielen der speziellen "Lagerung" der Führungsplatte 8 über den Lagerzapfen 11 bzw. die Lagerschraube 12 an der Lagerplatte 9 ist in einfacher Weise ein zu Reinigungszwecken erforderlicher Abstand der Kontaktflächen 22 und 23 der Führungsplatte 8 und der Lagerplatte 9 einstellbar, welcher unabhängig von der Schwenklage der Führungsplatte 8 zur Lagerplatte 9 erhalten bleibt. Insoweit ist unabhängig von der weiteren Handhabung bei Reinigungszwecken eine sichere Reinigung des erfindungsgemäßen medizinischen Instrumentes 1 durchführbar.

Dieses medizinische Instrument 1, welches beispielhaft als Schere dargestellt ist, kann auch in jeder anderen Art und Weise, beispielsweise auch als Zange, ausgebildet sein.

## Patentansprüche

1. Scherenartiges oder zangenartiges medizinisches Instrument (1) mit einem ersten und einem zweiten Handhebel (2, 3), welche jeweils ein Griffteil (6, 7) und ein Werkzeugteil (4, 5) aufweisen und welche über einen mit einem radial erweiterten Kopfteil (13) versehenen Lagerzapfen (11, 12) schwenkbar miteinander in Verbindung stehen, wobei der erste Handhebel (2) im Bereich zwischen seinem Griffteil (6) und seinem Werkzeugteil (4) eine Führungsplatte (8) bildet und der zweite Handhebel (3) im Bereich zwischen seinem Griffteil (7) und seinem Werkzeugteil (5) eine Lagerplatte (9) bildet, in deren Bereich der Lagerzapfen (11, 12) feststehend angeordnet ist, wobei die Führungsplatte (8) zur schwenkbaren Aufnahme des Lagerzapfens (11, 12) einen als sich in Längsrichtung der Führungsplatte (8) erstreckendes Langloch ausgebildeten Durchbruch (10) aufweist, wobei das Langloch (10) im Randbereich seines dem Werkzeugteil (4) benachbarten Endbereichs eine erste erweiterte Vertiefung (17) aufweist und wobei sich die Führungsplatte (8) und die Lagerplatte (9) im normalen Betriebszustand des Instrumentes (1) flächig berühren und, wobei die Lagerplatte (9) auf ihrer der Führungsplatte (8) zugewandten Kontaktfläche (23) eine im Bereich des Lagerzapfens (11, 12) angeordnete, quer zur Lagerplatte (9) verlaufende Quernut (20) aufweist, deren Tiefe (**T3**) zumindest der Tiefe **(T1)** der ersten Vertiefung (17) des Langloches (10) entspricht und deren Breite (**b**) zumindest der Breite (**B**) der Führungsplatte (8) entspricht und,
wobei der erste Handhebel (2) mit seiner Führungsplatte (8) in eine mit der Quernut (20) fluchtende Schwenkposition bringbar und in Richtung des Lagerzapfens (11, 12) in die Quernut (20) soweit verstellbar ist, dass das Kopfteil (13) des Lagerzapfens (11, 12) mit der ersten Vertiefung (17) des Langloches (10) außer Eingriff gelangt,
**dadurch gekennzeichnet,**
**dass** das Langloch (10) im Randbereich seines in Längsrichtung (18) dem Werkzeugteil (4) gegenüber liegenden Endbereichs eine zweite erweiterte Vertiefung (19) aufweist, und
**dass** das Kopfteil (13) des Lagerzapfens (11, 12) wahlweise in der ersten oder der zweiten Vertiefung (17 oder 19) aufnehmbar ist und,
**dass** die erste Vertiefung (17) eine geringere Tiefe (**T1**) aufweist als die zweite Vertiefung (19) und,
**dass** das Kopfteil (13) des Lagerzapfens (11, 12) durch eine Verstellung des zweiten Handhebels (3) mit seiner Lagerplatte (9) und dem Lagerzapfen (11, 12) entlang des Langloches (10) mit der zweiten Vertiefung (19) des Langloches (10) in Überdeckung und durch eine Verstellung in Richtung (24) des Lagerzapfens (11, 12) mit der zweiten Vertiefung (19) in Eingriff bringbar ist, ohne dass das Instrument (1) auseinander fallen kann und,
**dass** die Führungsplatte (8) und die Lagerplatte (9) in dieser Position einen Abstand voneinander aufweisen.

2. Instrument nach Anspruch 1, **dadurch gekennzeichnet, dass** die Tiefe (**T1**) der ersten Vertiefung (17) des Langloches (10) derart bemessen ist, dass das Kopfteil (13) in der normalen Betriebsstellung darin zumindest annähernd vollständig aufgenommen ist.

3. Instrument nach Anspruch 1 oder 2, **dadurch gekennzeichnet, dass** der Lagerzapfen (11) als Lagerschraube (12) ausgebildet ist, welche mit einem Gewindezapfen (15) in ein Innengewinde (16) der Lagerplatte (9) einschraubbar ist und,
dass das Innengewinde (16) zentral in der Quernut (20) der Lagerplatte (9) angeordnet ist.

4. Instrument nach einem der Ansprüche 1 bis 3, **dadurch gekennzeichnet, dass** das Langloch (10) der Führungsplatte (8) zwischen den beiden Vertiefungen (17, 19) im Bereich seiner Längskanten jeweils eine seitlich eingesenkt abgesetzte Erweiterung (35) aufweist, über welche die Vertiefungen (17, 19) miteinander in Verbindung stehen und,
dass die Vertiefungen (17, 19) mit einer größeren Tiefe (T1, **T2**) ausgestattet sind als die seitliche Erweiterung.

5. Instrument nach einem der Ansprüche 1 bis 4, **dadurch gekennzeichnet, dass** die Vertiefungen (17, 19) mit konisch zum Langloch (10) hin geneigt verlaufenden Grundflächen (36, 37) versehen sind und,
dass das Kopfteil (13) des Lagerzapfens (11, 12) unterseitig zum Langloch (10) hin eine umlaufende, konische Auflagefläche (38) bildet, mit welcher das Kopfteil (13) passend mit einer der Grundflächen (36 oder 37) in Eingriff bringbar ist.

6. Instrument nach einem der Ansprüche 1 bis 5, **dadurch gekennzeichnet, dass** zwischen der Lagerplatte (9) und der Führungsplatte (8) ein Federelement, insbesondere in Form einer Tellerfeder (32) vorgesehen ist, durch welche das Kopfteil (13) in seiner jeweiligen Eingriffsposition mit einer der beiden Vertiefungen (17 oder 19) des Langloches (10) gehalten ist.

7. Instrument nach Anspruch 6, **dadurch gekennzeichnet, dass** das Federelement (32) in einer um den Lagerzapfen (11, 12) der Lagerplatte (9) umlaufenden, ringförmigen Einsenkung (21) aufgenommen ist.

## Claims

1. Medical instrument (1) in the form of scissors or forceps, with a first and a second hand lever (2, 3) which each have a grip part (6, 7) and a tool part (4, 5) and which are pivotably connected to each other via a bearing pin (11, 12) that is provided with a radially enlarged head part (13), wherein the first hand lever (2), in the area between its grip part (6) and its tool part (4), forms a guide plate (8), and the second hand lever (3), in the area between its grip part (7) and its tool part (5), forms a bearing plate (9), in the area of which the bearing pin (11, 12) is fixedly arranged, wherein the guide plate (8), in order to pivotably receive the bearing pin (11, 12), has an aperture (10) designed as an oblong hole extending in the longitudinal direction of the guide plate (8), wherein the oblong hole (10) has a first enlarged depression (17) in the edge area of its end region adjacent to the tool part (4), and wherein the guide plate (8) and the bearing plate (9) are in planar contact with each other in the normal operating state of the instrument (1), and wherein the bearing plate (9), on its contact face (23) directed towards the guide plate (8), has a transverse groove (20) which is arranged in the area of the bearing pin (11, 12) and extends transversely with respect to the bearing plate (9), and of which the depth (T3) corresponds at least to the depth (T1) of the first depression (17) of the oblong hole (10), and of which the width (b) corresponds at least to the width (B) of the guide plate (8), and
wherein the first hand lever (2) can be brought with its guide plate (8) into a pivot position flush with the transverse groove (20) and can be moved in the direction of the bearing pin (11, 12) into the transverse groove (20), to such an extent that the head part (13) of the bearing pin (11, 12) disengages from the first depression (17) of the oblong hole (10),
**characterized in that**
the oblong hole (10), in the edge area of its end region lying opposite the tool part (4) in the longitudinal direction (18), has a second enlarged depression (19), and
the head part (13) of the bearing pin (11, 12) can be received alternatively in the first or the second depression (17 or 19), and
the first depression (17) has a lesser depth (T1) than the second depression (19), and
the head part (13) of the bearing pin (11, 12) can be brought to overlap the second depression (19) of the oblong hole (10), by an adjustment of the second hand lever (3) with its bearing plate (9) and the bearing pin (11, 12) along the oblong hole (10), and can be brought into engagement with the second depression (19) by an adjustment in the direction (24) of the bearing pin (11, 12), without the instrument (1) being able to fall apart, and
the guide plate (8) and the bearing plate (9) are spaced apart from each other in this position.

2. Instrument according to Claim 1, **characterized in that** the depth (T1) of the first depression (17) of the oblong hole (10) is dimensioned such that the head part (13) is received almost completely therein in the normal operating position.

3. Instrument according to Claim 1 or 2, **characterized in that** the bearing pin (11) is designed as a bearing screw (12), which can be screwed with a threaded pin (15) into an inner thread (16) of the bearing plate (9), and
the inner thread (16) is arranged centrally in the transverse groove (20) of the bearing plate (9).

4. Instrument according to one of Claims 1 to 3, **characterized in that** the oblong hole (10) of the guide plate (8) has, in the area of each of its longitudinal edges between the two depressions (17, 19), a laterally offset enlargement (35) via which the depressions (17, 19) are connected to each other, and
the depressions (17, 19) have a greater depth (T1, T2) than the lateral enlargement.

5. Instrument according to one of Claims 1 to 4, **characterized in that** the depressions (17, 19) are provided with bottom surfaces (36, 37) sloping conically towards the oblong hole (10), and
the head part (13) of the bearing pin (11, 12) forms, on the underside towards the oblong hole (10), a peripheral, conical support surface (38), with which the head part (13) can be brought snugly into engagement with one of the bottom surfaces (36 or 37).

6. Instrument according to one of Claims 1 to 5, **characterized in that** a spring element is provided between the bearing plate (9) and the guide plate (8), particularly in the form of a disc spring (32), by which the head part (13) is held in its respective position of engagement with one of the two depressions (17 or 19) of the oblong hole (10).

7. Instrument according to Claim 6, **characterized in that** the spring element (32) is received in an annular recess (21) extending about the bearing pin (11, 12) of the bearing plate (9).

## Revendications

1. Instrument médical en forme de ciseaux ou de pince (1) comprenant un premier et un deuxième levier manuel (2, 3), lesquels présentent à chaque fois une partie de préhension (6, 7) et une partie d'outil (4, 5) et lesquels sont en liaison pivotante l'un avec l'autre par le biais d'un tourillon de palier (11, 12) pourvu d'une partie de tête (13) élargie radialement, le premier levier manuel (2) formant, dans la région entre sa partie de préhension (6) et sa partie d'outil (4), une plaque de guidage (8), et le deuxième levier manuel (3) formant, dans la région entre sa partie de préhension (7) et sa partie d'outil (5), une plaque de palier (9) dans la région de laquelle le tourillon de palier (11, 12) est disposé fixement, la plaque de guidage (8) présentant, pour recevoir de manière pivotante le tourillon de palier (11, 12), un orifice (10) réalisé sous forme de trou oblong s'étendant dans la direction longitudinale de la plaque de guidage (8), le trou oblong (10) présentant, dans la région de bord de sa région d'extrémité adjacente à la partie d'outil (4), un premier renfoncement élargi (17) et la plaque de guidage (8) et la plaque de palier (9), dans l'état de fonctionnement normal de l'instrument (1), étant en contact à plat et la plaque de palier (9) présentant, sur sa face de contact (23) tournée vers la plaque de guidage (8), une rainure transversale (20) disposée dans la région du tourillon de palier (11, 12), s'étendant transversalement par rapport à la plaque de palier (9), dont la profondeur (T3) correspond au moins à la profondeur (T1) du premier renfoncement (17) du trou oblong (10) et dont la largeur (b) correspond au moins à la largeur (B) de la plaque de guidage (8), et
le premier levier manuel (2) pouvant être amené avec sa plaque de guidage (8) dans une position de pivotement en affleurement avec la rainure transversale (20) et pouvant être déplacé dans la direction du tourillon de palier (11, 12) dans la rainure transversale (20) dans une mesure telle que la partie de tête (13) du tourillon de palier (11, 12) parvienne hors d'engagement avec le premier renfoncement (17) du trou oblong (10), **caractérisé en ce que**
le trou oblong (10), dans la région de bord de sa région d'extrémité opposée dans la direction longitudinale (18) à la partie d'outil (4), présente un deuxième renfoncement élargi (19), et la partie de tête (13) du tourillon de palier (11, 12) peut être reçue de manière sélective dans le premier ou le deuxième renfoncement (17 ou 19), et le premier renfoncement (17) présente une plus faible profondeur (T1) que le deuxième renfoncement (19) et
la partie de tête (13) du tourillon de palier (11, 12) peut, par un déplacement du deuxième levier manuel (3) avec sa plaque de palier (9) et avec le tourillon de palier (11, 12) le long du trou oblong (10), être amenée à coïncider avec le deuxième renfoncement (19) du trou oblong (10) et, par un déplacement dans la direction (24) du tourillon de palier (11, 12), être amenée en prise avec le deuxième renfoncement (19), sans que l'instrument (1) ne puisse se désassembler, et
la plaque de guidage (8) et la plaque de palier (9) présentent dans cette position un espacement l'une par rapport à l'autre.

2. Instrument selon la revendication 1, **caractérisé en ce que** la profondeur (T1) du premier renfoncement (17) du trou oblong (10) est dimensionnée de telle sorte que la partie de tête (13) soit reçue dans la position de fonctionnement normal au moins approximativement complètement dans celui-ci.

3. Instrument selon la revendication 1 ou 2, **caractérisé en ce que** le tourillon de palier (11) est réalisé sous forme de vis de palier (12) qui peut être vissée avec un tourillon fileté (15) dans un filetage interne (16) de la plaque de palier (9) et
le filetage interne (16) est disposé centralement dans la rainure transversale (20) de la plaque de palier (9).

4. Instrument selon l'une quelconque des revendications 1 à 3, **caractérisé en ce que** le trou oblong (10) de la plaque de guidage (8) présente, entre les deux renfoncements (17, 19) dans la région de ses arêtes longitudinales, à chaque fois un élargissement (35) en retrait renfoncé latéralement, par le biais duquel les renfoncements (17, 19) sont en liaison l'un avec l'autre et
les renfoncements (17, 19) sont réalisés avec une plus grande profondeur (T1, T2) que l'élargissement latéral.

5. Instrument selon l'une quelconque des revendications 1 à 4, **caractérisé en ce que** les renfoncements (17, 19) sont pourvus de surfaces de base (36, 37) inclinées coniquement vers le trou oblong (10) et
la partie de tête (13) du tourillon de palier (11, 12) forme, du côté inférieur vers le trou oblong (10), une surface d'appui conique périphérique (38) avec laquelle la partie de tête (13) peut être amenée en prise de manière ajustée avec l'une des surfaces de base (36 ou 37).

6. Instrument selon l'une quelconque des revendications 1 à 5, **caractérisé en ce qu'**entre la plaque de palier (9) et la plaque de guidage (8) est prévu un élément de ressort, en particulier sous forme de ressort à coupelle (32), par lequel la partie de tête (13) est retenue dans sa position d'engagement respective avec l'un des deux renfoncements (17 ou 19) du trou oblong (10).

7. Instrument selon la revendication 6, **caractérisé en ce que** l'élément de ressort (32) est reçu dans une dépression (21) de forme annulaire s'étendant tout autour du tourillon de palier (11, 12) de la plaque de palier (9).
